Europäisches Patentamt

European Patent Office

Office européen des brevets

(11) Publication number: **0 514 939 B1**

# EUROPEAN PATENT SPECIFICATION

(45) Date of publication of patent specification: **12.10.94**   (51) Int. Cl.5: **C07K 5/06**

(21) Application number: **92108707.8**

(22) Date of filing: **22.05.92**

(54) Method of preparing alpha-L-aspartyl-L-phenylalanine methyl ester.

(30) Priority: **23.05.91 JP 221335/91**

(43) Date of publication of application:
**25.11.92 Bulletin 92/48**

(45) Publication of the grant of the patent:
**12.10.94 Bulletin 94/41**

(84) Designated Contracting States:
**BE CH DE FR GB IT LI NL**

(56) References cited:
**EP-A- 0 128 694**
**EP-A- 0 405 273**

**CA 109:211484,corresponding to JP
63-145298 (Mitsui Toatsu)**

(73) Proprietor: **Ajinomoto Co., Inc.
No. 15-1, Kyobashi 1-chome
Chuo-ku
Tokyo (JP)**

(72) Inventor: **Kishimoto, Shinichi c/o
Cent.Res.Lab.
Ajinomoto Co.,Inc.,
No.1-1 Suzuki-cho,
Kawasaki-ku
Kawasaki-shi, Kanagawa-ken (JP)**
Inventor: **Abe, So, c/o Cent.Res.Lab.
Ajinomoto Co.,Inc.,
No.1-1 Suzuki-cho,
Kawasaki-ku
Kawasaki-shi, Kanagawa-ken (JP)**
Inventor: **Kato, Toshihisa, c/o Cent.Res.Lab.
Ajinomoto Co.,Inc.,
No.1-1 Suzuki-cho,
Kawasaki-ku
Kawasaki-shi, Kanagawa-ken (JP)**

(74) Representative: **Strehl Schübel-Hopf Groening
& Partner
Maximilianstrasse 54
D-80538 München (DE)**

## Description

The present invention relates to a method of preparing α-L-aspartyl-L-phenylalanine methyl ester (hereinafter referred to as "α-APM") which is useful as a sweetener, specifically it relates to an extremely improved method of preparing α-APM by neutralizing an acid addition salt of α-APM with a base.

α-APM of the present invention is a dipeptide sweetener having a sweetness of about 200 times that of sucrose (cane sugar). Because of the extremely good sweetening properties and the low caloric value, it has become used significantly as a diet sweetener in these days, and the worldwide demand for it is presumed to be over 10,000 tons before 1995.

For industrial production of α-APM, for example, the following methods are known. Precisely, they are (1) a method of obtaining α-APM in which an N-substituted aspartic acid anhydride and a phenylalanine methyl ester are bonded to each other in an organic solvent and the substituent is removed from the product by an ordinary method (U.S. Patent 3,786,039); (2) a method of obtaining α-APM in which α-L-aspartyl-L-phenylalanine is methyl-esterified in a mixed solvent comprising water, methanol and hydrochloric acid to obtain α-APM hydrochloride, and this is neutralized to obtain α-APM (Japanese Patent Application Laid-Open No. 53-82752); and (3) a method of obtaining α-APM in which an N-substituted aspartic acid and a phenylalanine methyl ester are condensed in the presence of an enzyme and thereafter the substituent is removed from the product (Japanese Patent Application Laid-Open No. 55-135595).

In the above-mentioned chemical synthetic method (1), a β-isomer (β-L-aspartyl-L-phenylalanine methyl ester) is produced essentially as a side product. As a means of selectively removing impurities including said β-isomer, there is known (4) a purification method in which α-APM containing impurities is brought into contact with a hydrohalogenic acid and then subjected to solid-liquid separation so as to isolate α-APM as its hydrohalide salt.

Where industrial scale production of α-APM is taken into consideration so as to meet the trend of the demands of the current time, chemical methods are the major methods from the viewpoint of the manufacture costs. In the case, esterification is often effected via its hydrochloride as in the above-mentioned method (2), or after the formation of α-APM, it is often formed into its hydrohalide salt such as the hydrochloride for purifying it as in method (4). For obtaining α-APM from its hydrohalide salt such as its hydrochloride by an ordinary method, a hydrohalide salt of α-APM is dissolved or suspended in an aqueous medium and the resulting solution or suspension is neutralized by adding an aqueous solution of a base such as sodium hydroxide, sodium hydrogen carbonate or ammonia.

However, the present inventors found that when a large amount of a liquid in a liter scale or more must be dealt with especially in the industrial scale neutralization of such an acid addition salt or α-APM, the conventional neutralization method has serious problems such as those mentioned below.

Precisely, where a base is continuously dropwise added to an aqueous solution of an acid addition salt of α-APM to the isoelectric point of α-APM, rapid precipitation of α-APM will occur during the course of the addition so that stirring of the system will become impossible. In the worst case, it has been found that the stirrer stops. If the liquid amount is at most 100 ml or so in a laboratory scale experiment, the precipitated solid phase may easily be broken with a tool such as a spatula by an experimentalist whereby the fluid condition may well be recovered. However, where a large amount of a liquid in a liter scale or more is used, for example, in a so-called bench plant or the like, or where the neutralization is carried out in a large-scaled pilot plant or commercial plant, such a means cannot be used for solving the problem.

As a countermeasure to the problem in the industrial scale neutralization, addition of a large amount of water may be considered so as to carry out the neutralization under diluted concentration conditions. However, this causes an extreme lowering of the capacity and efficiency of the device used and also causes an extreme lowering of the yield of the product. Therefore, the means are not satisfactory. On the other hand, extremely slow addition of an aqueous base solution over an extremely long period of time will be effective for ensuring the fluidity of the liquid. This method, however, is extremely ineffective from the viewpoint of the producibility. In addition, still another method of intermittently discontinuing the dropwise addition of the neutralizing agent to the reaction system having a pH range at which precipitation of α-APM starts or a pH value being 2.5 or so to thereby ripen the precipitated crystals may be employed (Japanese Patent Application Laid-Open No. 63-145298). However, the method also has the serious drawback that the pH range suitable for the ripening will greatly fluctuate unless the initial concentration of α-APM (or its acid addition salt such as its hydrochloride) is strictly controlled to a certain value. It is always the case that the α-APM content in the separated wet crystals (acid addition salt) fluctuates depending upon the delicate conditions in crystallizing the acid addition salt of α-APM in the previous step. In such a case. It is difficult to keep the initial concentration of α-APM (or its acid addition salt such as its hydrochloride) constant in a dissolution system to be operated under control of the liquid amount which is often employed in industrial production. In order to evade the problem, a complicated concentration control is necessary to effect batchwise analysis every time to supply crystals or water, when needed; or an expert monitor who is skilled in the art must be exclusively engaged to the system so as to be able to batchwise determine the suitable ripening pH value every time.

In addition, even though the problems of the operation could be evaded by any of the above-mentioned systems, the α-APM crystals to be obtained still have an extremely poor solid-liquid separatability so that the crystals cause a noticeable increase of the necessary equipment and energy in the filtration and drying step as the post-treatment steps.

The present inventors made earnest and repeated investigations for the purpose of overcoming the above-mentioned problems in the neutralization step of neutralizing an acid addition salt of α-APM and, as a result, have obtained the following findings.

Specifically, an acid addition salt of α-APM is first dissolved or suspended in an aqueous medium to have a concentration of 3 % or more, as a liquid A, and it is maintained to be an acidic solution having a pH of 3 or less. On the other hand, a liquid containing or not containing α-APM is prepared as a solution B. With stirring, solution A is gradually added to solution B with optionally adding a base substance thereto, whereupon the pH of the resulting liquid blend is kept to be 3 or more so that α-APM crystals are crystallized out. In such a way, they have found that the above-mentioned problems of the operation and filtration can be overcome without necessity of any skill and that the yield of the intended product is higher than that to be obtained by the conventional methods.

Obtaining the findings, the present inventors have applied them to an actual process of industrial production of α-APM and have attained drastic rationalization of the process of producing α-APM, including reduction of the number of the members necessary for carrying out the process. Thus, they have hereby completed the present invention.

Acid addition salts of α-APM usable in the present invention may be mineral acid salts such as the hydrochloride hydrobromide, sulfate and phosphate of it. Especially, the hydrochloride is often used.

In the present invention, an acid addition salt of α-APM is neutralized in the form of an aqueous solution or suspension. In this case, all of the acid addition salt crystals need not to be dissolved. As the solvent, water or a mixed solvent comprising a water-miscible organic solvent and water are suitable. The amount of the solvent to be used may be determined in such a way that the concentration of the acid addition salt of α-APM in the resulting solution or suspension is 3 % or more. This is because if the concentration is lower than the determined value, the yield and the producibility per the device capacity will lower and the filterability of the crystals to be precipitated out after neutralization will unfavorably rapidly decrease. The upper limit of the concentration is not specifically defined, and it may well be determined suitably in view of the system of the apparatus to be used, such as the pipe line, the pump or the like, or of the operability of the process. As a general range, the concentration is suitably from 3 to 25 %.

Blending and neutralization must be effected gradually, in carrying out the method of the present invention, so that the solution or suspension of an acid addition salt of α-APM is kept as it is during the process. However, α-APM dissolved in an aqueous medium under low pH condition is hydrolyzed to give α-L-aspartyl-L-phenylalanine (hereinafter referred to as " α-AP"). Such decomposition reaction will lower the yield and quality of the product of the present invention and is therefore unfavorable in production of the product. As the decomposition often occurs especially at a high temperature, the temperature of the reaction system must be 50 ° C or lower, more preferably 40 ° C or lower.

Where wet α-APM-acid addition salt crystals just as separated in the previous acid addition salt crystallizing step are used, the solution or suspension containing the acid addition salt has a pH value further lowered due to the excess acid in the mother solution as adhered to the wet crystals so that the decomposition will be accelerated. In order to prevent the accelerated decomposition, partial neutralization of the wet crystals and storage of the thus partially neutralized crystals are desired. In this case, a suitable pH range is from 2 to 3, desirably from 2.3 to 2.8. Precipitation of α-APM of such a degree that would not interfere with the subsequent operation would cause no problem in carrying out the present invention. However, if the pH value is more than 3, rapid precipitation of α-APM crystals will occur. Therefore, the partial neutralization is to be effected at the pH value not overstepping the defined range.

Bases, suitable as a neutralizing agent are alkali hydroxides such as sodium hydroxide, alkali carbonates or bicarbonates such as sodium carbonate, ammonia and other organic amines. From the viewpoint of prevention of decomposition of α-APM during neutralization as well as of the costs and the easy operability, sodium carbonate or ammonia is frequently used in the form of its aqueous solution.

The other liquid to be used for blending is one containing or not containing α-APM as dissolved or suspended therein prior to blending. For instance, if not the entire amount of the suspension of α-APM crystals obtained by the method of the present invention is subjected to solid-liquid separation, a part of it may be used in the next blending and neutralization batch; or alternatively, only the mother solution to be separated by the solid-liquid separation may be used again in the next batch. However, in order to moderate rapid precipitation of α-APM crystals by neutralization, the presence of α-APM crystals as seed crystals in the liquid is more preferred.

The amount of the liquid must be determined in such a way that the liquid blend may have a final α-APM concentration not lower than 3 % after blending and neutralization for the purpose of improving the solid-liquid separability and of elevating the yield, especially when the liquid does not contain α-APM. The liquid may previously contain a basic substance necessary for neutralization. However, α-APM dissolved in an aqueous medium would easily form a diketopiperazine compound (hereinafter referred to as DKP) under high pH conditions due to intramolecular cyclization reaction. Even though the liquid contains no α-APM before blending, it is desired that a suitable amount of a basic substance, which is well proportional to the addition speed of the acidic solution thereto is gradually supplied to the liquid in consideration of the situation just after the initiation of blending.

Blending is effected with stirring so that local distribution of pH, temperature and concentration in the reaction system may be minimized to the least. Stirring as referred to herein indicates to cause a forced flow by stirring blades, pump circulation or introduction of air bubbles, and it needs a strength sufficient for at least floating and suspending the crystals existing in the system in the liquid to form a so-called slurry during the course of from the start of blending and neutralization to the end thereof.

The pH value of the liquid blend must be maintained to be always 3 or more. The upper limit is desirably 6 from the above-mentioned viewpoint of prevention of decomposition. The final pH value is desirably from 4 to 6. The liquid temperature is to be 40 °C or lower from the viewpoint of prevention or decomposition at a high temperature and of improvement of the yield. The lower limit need not be determined specifically, provided that it is such that the solvent is not frozen. In general, the lower limit is determined to fall within the range of from 5 °C to 30 °C. The addition speed of the solution or suspension of an α-APM acid addition salt varies, in accordance with the scale (liquid amount) of the process to be carried out. It may have any desired value as long as it avoids any rapid neutralization or precipitation of crystals to result in some operation troubles.

The blending and neutralization may be effected in one container in carrying out the method of the present invention, but, if desired, stepwise blending and neutralization and optionally cooling may be effected with plural containers connected to each other in series. Where the latter system of having plural containers is used, precipitation load of α-APM may be dispersed in plural containers extremely effectively from the viewpoint of improvement of the operation. The process of the present invention drastically reduces the necessary equipment and energy costs and reduces the number of personnel necessary for carrying out the neutralization and crystallization process.

In accordance with the method of the present invention for preparing α-APM by neutralizing an acid addition salt of α-APM with a base in an industrial scale, especially in a liter scale or larger, the operability of the liquid such as the fluidity of the liquid during neutralization is extremely improved and the solid-liquid separability of the α-APM crystals to be produced as well as the yield thereof is noticeably improved. Additionally, the steps constituting the method are simplified. Therefore, the method of the present invention is extremely valuable in industrial use.

Examples:

Next, the present invention will be explained in more detail by way of the following examples.

The test of evaluating the filterability of α-APM crystals as obtained in the examples was effected by the method mentioned below.

Method of Measuring Filtration Specific Cake Resistance

One liter of a sample to be tested is sampled and is filtered through a top-feed system suction filter (leaf tester). The pressure difference in filtration is 70 mmHg, which is kept constant throughout the period of filtration. From the start of filtration, the amount of the filtrate $V[cm^3]$ is measured at regular intervals and plotted on a graph having the amount of the filtrate as the horizontal axis and the value $(\theta/V)$ obtained by dividing time of filtration $\theta$-[s] by the amount of the filtrate as the vertical axis. The inclination of the line $K[s/ml^2]$ is obtained by the minimum square method. The value C' as obtained by dividing the total amount [g] of the crystals in the slurry by the total amount $[cm^3]$ of the liquid in the slurry is introduced into the following equation. The filtration area A is 93 [cm$^2$]; and the viscosity $\mu$ of the filtrate is 0.0135 [g/cm•s]. The specific cake resistance $\alpha$ thus calculated is a criterion for the filterability of the sample. A sample having a smaller value $\alpha$ is more easily filterable.

Equation of Specific Cake Resistance.

$$\alpha = 20.K.A^2.PT/ \mu.C' \ [m/kg]$$

where $\alpha$ is the specific cake resistance [m/kg] of the filtered cake;

$\mu$ is the viscosity of the filtrate [g/cm.s] ;

PT is the pressure difference [dyne/cm$^2$] by the filtered cake and the filtration device = $\Delta P$-[mmHg] $\times$ 1333.22;

A is filtration area [cm $^2$]; and

C' is the weight of the crystals per the unit volume of the liquid component in the slurry [g/cm$^3$] = dry cake weight [g]/(wet cake weight [g] - dry cake weight [g] + amount of final filtrate [cm$^3$]).

Example 1:

110.8 g of wet crystals of $\alpha$-APM hydrochloride (containing 62 % of $\alpha$-APM) were dissolved in 1400 ml of water at 28 ° C, which was adjusted to have a pH of 2.5 with 16 % sodium carbonate to prepare an $\alpha$-APM hydrochloride solution. This was dropwise added to a suspension of 25 g of $\alpha$-APM as suspended in 500 ml of water of 3.5 ° C, in a 2.5-liter jacket-combined separable flask around which cold water of 3.5 ° C was circulated, at a constant addition rate with stirring with an anchor-shaped stirring blade having a blade diameter of 10 cm, at 200 rpm, over a period of 3 hours. During the addition, the temperature of the solution was adjusted to be from 3.5 to 5.0 ° C and the pH of the same to be always 5.0 with 16 % sodium carbonate. During the addition, rapid precipitation of crystals was not admitted and the slurry kept good fluidity. After the addition, the slurry obtained was subjected to the leaf test to obtain a result of a good value of 9.8 $\times$ 10$^9$ [m/kg]. The dry yield was 85.3 g (90.9 %).

Example 2:

2358 g of wet crystals of $\alpha$-APM hydrochloride were dissolved in 23 liters of water at 30 ° C (6 g/dl as $\alpha$-APM concentration), which was adjusted to have a pH of 2.5 with 10 % sodium carbonate to prepare an $\alpha$-APM hydrochloride solution. This was dropwise added to a jacket-combined flask (with stirring at 200 rpm) to which a coolant of 5 ° C was applied, at a constant rate of 15 ml/min. The flask contained the slurry as prepared by the method of Example 1 and was adjusted to have pH of 4.8, as a receiving liquid. During the dropwise addition, the liquid amount in the flask is always maintained to be 5 liters by allowing the excessive liquid/slurry to overflow, and the pH of the liquid in the flask was adjusted to be always 4.8 by dropwise adding 10 % sodium carbonate thereto at a rate of 1.25 ml/min. The concentration of the solution was adjusted to always have an $\alpha$-APM concentration of 4.8 g/dl by adding an $\alpha$-APM solution of 5 ° C ($\alpha$-APM concentration 0.6 g/dl) at a rate of from 2.7 to 3.1 ml/min. The continuous operation was effected for 24 hours, whereupon rapid precipitation of crystals was not admitted and the slurry kept good fluidity. The overflown slurry was received in a container as cooled with ice and was continuously stirred. The slurry as overflown and received in the container in the last one hour was subjected to the leaf test to obtain a result of 3.0 $\times$ 10$^{10}$ [m/kg].

Comparative Example 1:

240 g of wet crystals of $\alpha$-APM hydrochloride (containing 62 % of $\alpha$-APM) were dissolved in 1900 ml of water at 28 ° C in a 2.5-liter jacket-combined separable flask around which a warm water of 30 ° C was circulated, to prepare an $\alpha$-APM hydrochloride solution. While the solution was kept to have a temperature of 28 ° C, 16 % sodium carbonate was dropwise added thereto at a constant rate (7 ml/min). The scheduled period before the completion of the neutralization is 30 minutes. Stirring was effected at 70 rpm with an anchor-shaped stirring blade having a blade diameter of 10 cm, but the liquid lost the fluidity with precipitation of crystals (pH 3.0, after 16 minutes), and the stirring stopped 21 minutes after the start of the experiment. The content in the flask was solidified.

Comparative Example 2:

150.3 g of wet crystals of $\alpha$-APM hydrochloride ( containing 62 % of $\alpha$-APM) were dissolved in 1900 ml of water at 28 ° C in a 2.5-liter jacket-combined separable flask around which warm water of 30 ° C was circulated, to prepare an $\alpha$-APM hydrochloride solution. While the solution was kept to have a temperature of 28 ° C, 16 % sodium carbonate was dropwise added thereto at a constant rate over a period of 6 hours until the pH value of the solution became 5 (21.7 ml/h). Stirring was effected at 200 rpm with an anchor-shaped stirring blade having a blade diameter of 10 cm, but the surface part of the liquid lost the fluidity with precipitation of crystals (pH 3.4) so that smooth operation could no more be conducted. The stirring rate was changed to 300 rpm in the course of the process. After completion of the addition, the system was cooled to 5 ° C over a period of 3 hours, and the slurry obtained was subjected to the leaf test. The result was $1.0 \times 10^{11}$ [m/kg]. The dry yield was 83.9 g (86 %).

Comparative Example 3:

100.2 g of wet crystals of $\alpha$-APM hydrochloride (containing 62 % of $\alpha$-APM) were dissolved in 1900 ml of water at 28 ° C in a 2.5-liter jacket-combined separable flask around which warm water of 30 ° C was circulated, to prepare an $\alpha$-APM hydrochloride solution. While the solution was kept to have a temperature of 28 ° C, 16 % sodium carbonate was dropwise added thereto at a constant rate over a period of 4 hours and 40 minutes until the pH value of the solution became 5 (19.6 ml/h). Stirring was effected at 200 rpm with an anchor-shaped stirring blade having a blade diameter of 8 cm. Even after precipitation of crystals, the content in the flask still kept the fluidity. After completion of the addition, the system was cooled to 5 ° C over a period of 2 hours, and the slurry obtained was subjected to the leaf test. The result was $1.08 \times 10^{11}$ [m/kg]. The dry yield was 52.37 g (84 %).

**Claims**

1. A method for preparing $\alpha$-L-aspartyl-L-phenylalanine methyl ester by neutralizing an acid addition salt of $\alpha$-L-aspartyl-L-phenylalanine methyl ester with a base, characterised in that

    (a) an acid addition salt of $\alpha$-L-aspartyl-L-phenylalanine methyl ester is dissolved or suspended in an aqueous medium in a concentration of said salt of 3% or more, and the resulting liquid is maintained at a pH of 3 or less and at a temperature of 50 °C or lower,

    (b) the liquid is gradually added to and blended with an aqueous medium with stirring, while optionally adding a basic substance thereto, said aqueous medium optionally containing $\alpha$-L-aspartyl-L-phenylalanine methyl ester, and

    (c) the resulting blend is kept at a temperature of 40 °C or lower and a pH of 3 or more so that crystals of $\alpha$-L-aspartyl-L-phenylalanine methyl ester are crystallised out.

2. The method according to claim 1, wherein an acid addition salt of $\alpha$-L-aspartyl-L-phenylalanine methyl ester is dissolved or suspended in an aqueous medium in a concentration of the salt of 3% or more and the pH of the resulting liquid is controlled to a range of 2 to 3 by adding a base thereto.

3. The method according to claim 1 or claim 2, wherein in step (b) a base is added simultaneously with the blending of the two liquids to control the pH of the liquid blend to be 3 to 6.

4. The method according to any of the claims 1 to 3, wherein the final pH of the liquid blend in step (c) is adjusted to be within the range of from 4 to 6.

5. The method according to any of the claims 1 to 4, in which the liquid blend is cooled to 30 °C simultaneously with or after blending of the two liquids.

6. The method according to any of the claims 1 to 5, wherein the mixing, neutralizing and/or cooling are/is carried out stepwise in a plurality of reaction containers.

7. The method according to any of the claims 1 to 6, wherein the acid addition salt of $\alpha$-L-aspartyl-L-phenylalanine methyl ester is the hydrochloride salt.

**Patentansprüche**

1. Verfahren zur Herstellung von $\alpha$-L-Aspartyl-L-phenylalanin-methylester durch Neutralisation eines Säureadditionssalzes von $\alpha$-L-Aspartyl-L-phenylalanin-methylester mit einer Base, dadurch gekennzeichnet, daß

    (a) ein säureadditionssalz von $\alpha$-L-Aspartyl-L-phenylalaninmethylester in einer Konzentration des Salzes von 3 % oder mehr in einem wäßrigen Medium gelöst oder suspendiert wird, und die gebildete Flüssigkeit bei einem pH-Wert von 3 oder weniger und einer Temperatur von 50 °C oder darunter gehalten wird,

    (b) die Flüssigkeit unter Rühren allmählich zu einem wäßrigen Medium gegeben wird und mit diesem vermischt wird, während gegebenenfalls eine basische Substanz zugefügt wird, wobei dieses wäßrige Medium gegebenenfalls $\alpha$-L-Aspartyl-L-phenylalanin-methylester enthält, und

    (c) das gebildete Gemisch bei einer Temperatur von 40 °C oder weniger und einem pH-Wert von 3 oder mehr gehalten wird, sodaß Kristalle von $\alpha$-L-Aspartyl-L-phenyla-lanin-methylester auskristallisieren.

2. Verfahren nach Anspruch 1, bei dem ein Säureadditionssalz von $\alpha$-L-Aspartyl-L-phenylala-nin-methylester in einer Konzentration des Salzes von 3 % oder mehr in einem wäßrigen Medium gelöst oder suspendiert wird und der pH-Wert der gebildeten Flüssigkeit durch Zugabe einer Base auf einen Bereich von 2 bis 3 eingestellt wird.

3. Verfahren nach Anspruch 1 oder Anspruch 2, bei dem in Stufe (b) eine Base gleichzeitig mit dem Mischen der beiden Flüssigkeiten zugefügt wird, um den pH-Wert des flüssigen Gemisches auf 3 bis 6 einzustellen.

4. Verfahren nach einem der Ansprüche 1 bis 3, bei dem der endgültige pH-Wert des flüssigen Gemisches in Stufe (c) so eingestellt wird, daß er im Bereich von 4 bis 6 liegt.

5. Verfahren nach einem der Ansprüche 1 bis 4, bei dem das flüssige Gemisch zum Zeitpunkt des Vermischens der beiden Flüssigkeiten oder danach auf 30 °C abgekühlt wird.

6. Verfahren nach einem der Ansprüche 1 bis 5, bei dem das Vermischen, die Neutralisation und/oder das Kühlen stufenweise in mehreren Reaktionsbehältern durchgeführt wird bzw. werden.

7. Verfahren nach einem der Ansprüche 1 bis 6, wobei das Säureadditionssalz des α-L-Aspartyl-L-phenylalanin-methylesters das Hydrochlorid ist.

## Revendications

1. Procédé de préparation d'ester méthylique d'α-L-aspartyl-L-phénylalanine par neutralisation d'un sel d'addition acide d'ester méthylique d'α-L-aspartyl-L-phénylalanine par une base, caractérisé en ce que :

   (a) on dissout ou on met en suspension dans un milieu aqueux un sel d'addition acide d'ester méthylique d'α-L-aspartyl-L-phénylalanine pour une concentration dudit sel supérieure ou égale à 3% et on maintient le liquide résultant à un pH inférieur ou égal à 3 et à une température inférieure ou égale à 50 °C,

   (b) on ajoute progressivement le liquide et on le mélange à un milieu aqueux en agitant, tout en ajoutant éventuellement une substance basique, ledit milieu aqueux contenant éventuellement l'ester méthylique d'α-L-aspartyl-L-phénylalanine et

   (c) on conserve le mélange résultant à une température inférieure ou égale à 40 °C et à un pH supérieur ou égal à 3 de sorte que des cristaux d'ester méthylique d'α-L-aspartyl-L-phénylalanine se séparent par cristallisation.

2. Procédé selon la revendication 1, dans lequel on dissout ou on met en suspension dans un milieu aqueux un sel d'addition acide d'ester méthylique d'α-L-aspartyl-L-phénylalanine pour une concentration du sel supérieure ou égale à 3 % et dans lequel on amène le pH du liquide résultant de façon contrôlée pour qu'il soit compris entre 2 et 3 en ajoutant une base.

3. Procédé selon la revendication 1 ou la revendication 2, dans lequel au cours de l'étape (b) on ajoute simultanément une base au mélange des deux liquides pour amener le pH du mélange de liquides de façon contrôlée entre 3 et 6.

4. Procédé selon l'une quelconque des revendications 1 à 3, dans lequel on ajuste le pH final du mélange de liquides au cours de l'étape (c) pour qu'il soit compris entre 4 et 6.

5. Procédé selon l'une quelconque des revendications 1 à 4, dans lequel on refroidit le mélange de liquides à 30 °C pendant ou après le mélange des deux liquides.

6. Procédé selon l'une quelconque des revendications 1 à 5, dans lequel on met en oeuvre par étapes le mélange, la neutralisation et/ou le refroidissement dans plusieurs récipients de réaction.

7. Procédé selon l'une quelconque des revendications 1 à 6, dans lequel le sel d'addition acide d'ester méthylique d'α-L-aspartyl-L-phénylalanine est le sel de type chlorhydrate.